(19) Europäisches Patentamt
European Patent Office
Office européen des brevets

(11) Veröffentlichungsnummer : **0 365 903 B1**

# (12) EUROPÄISCHE PATENTSCHRIFT

(45) Veröffentlichungstag der Patentschrift : **02.12.92 Patentblatt 92/49**

(51) Int. Cl.⁵ : **C07C 271/20,** C08F 220/36, C08G 18/67, C08G 18/72, C08G 18/75, C09D 133/10

(21) Anmeldenummer : **89118694.2**

(22) Anmeldetag : **07.10.89**

## (54) Olefinisch ungesättigte Verbindungen, ein Verfahren zur Herstellung von Isocyanatgruppen aufweisenden Copolymerisaten, die so erhaltenen Copolymerisate und die Copolymerisate enthaltende Beschichtungsmittel bzw. Dichtmassen.

(30) Priorität : **21.10.88 DE 3835935**

(43) Veröffentlichungstag der Anmeldung : **02.05.90 Patentblatt 90/18**

(45) Bekanntmachung des Hinweises auf die Patenterteilung : **02.12.92 Patentblatt 92/49**

(84) Benannte Vertragsstaaten : **AT BE CH DE ES FR GB IT LI NL SE**

(56) Entgegenhaltungen :
**EP-A- 0 206 217**
**EP-A- 0 207 257**
**EP-A- 0 278 351**
**DE-A- 3 743 996**
**FR-A- 2 450 264**
**US-A- 2 958 704**
**US-A- 3 789 965**

(73) Patentinhaber : **BAYER AG**
**W-5090 Leverkusen 1 Bayerwerk (DE)**

(72) Erfinder : **Wamprecht, Christian, Dr.**
**Bahnhofstrasse 51**
**W-4040 Neuss 22 (DE)**
Erfinder : **Blum, Harald, Dr.**
**Auf dem Westkamp 1**
**W-4175 Wachtendonk 1 (DE)**
Erfinder : **Pedain, Josef, Dr.**
**Haferkamp 6**
**W-5000 Köln 80 (DE)**
Erfinder : **Halpaap, Reinhard, Dr.**
**Kleinfeldchensweg 37b**
**W-5000 Köln 91 (DE)**
Erfinder : **Klein, Gerhard, Dr.**
**Von-Flotow-Strasse 7**
**W-4019 Monheim (DE)**
Erfinder : **Arlt, Dieter, Prof. Dr.**
**Rybnikerstrasse 2**
**W-5000 Köln 80 (DE)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft olefinisch ungesättigte Verbindungen mit an tertiäre Kohlenstoffatome gebundenen Isocyanatgruppen, ein Verfahren zur Herstellung von Copolymerisaten unter Verwendung dieser Verbindungen, die entsprechenden Copolymerisate und Beschichtungsmittel bzw. Dichtmassen, die als Bindemittelkomponente derartige Copolymerisate enthalten.

Copolymerisate mit freien Isocyanatgruppen und deren Verwendung als Bindemittelkomponente in Zweikomponenten-Beschichtungsmassen sind bekannt.

So werden beispielsweise in DE-OS 3 245 294, 3 245 295 und 3 245 298 isocyanatfunktionelle Copolymerisate beschrieben, die durch Verwendung von Vinylisocyanat als ungesättigtes NCO-funktionelles Monomer hergestellt wurden, während in den US-PS 3 004 527, 4 219 632, 4 401 794 und 4 510 298 isocyanatfunktionelle Copolymerisate beschrieben werden, die durch Verwendung von Isocyanatoethylmethacrylat als NCO-funktionelles Monomer hergestellt wurden. Bei Verwendung dieser beiden NCO-funktionellen Monomeren zur Herstellung von Copolymerisaten werden Copolymerisate erhalten, die primäre Isocyanatgruppen aufweisen. Diese primären Isocyanatgruppen sind jedoch gegenüber häufig verwendeten stickstoffhaltigen Vernetzersubstanzen wie z.B. Polyketimine oder Polyoxazolane zu reaktionsfähig, so daß bei der Anwendung als Zweikomponentenbeschichtungssysteme keine ausreichend lange Topfzeiten erzielt werden.

Copolymerisate mit chemisch eingebauten tert. aliphatischen Isocyanatgruppen werden beispielsweise in EP-A-0 130 322, EP-A-0 130 323, J 61 120-667-A (C.A. 105 192 987), J 61 120-862-A (C.A. 105 192 967), J 61 126-177-A (C.A. 105 192 991) oder J 61 126-178-A (C.A. 105 192 992) beschrieben. Hierbei erfolgt die Einführung der Isocyanatgruppe durch die Verwendung vom m-Isopropenyl-α,α-dimethylbenzylisocyanat als isocyanatfunktionelles copolymerisierbares Monomer. Bei Verwendung dieses Monomers zur radikalischen Copolymerisation mit anderen ungesättigten Monomeren wie z.B. Estern der (Meth)acrylsäure oder Styrol findet man jedoch immer größere Mengen an Restmonomeren, weil die quasi α-Methylstyrolstruktur des m-Isopropenyl-α,α-dimethylbenzylisocyanats aufgrund ungünstiger Copolymerisationsparameter nur ungenügend mit den obengenannten anderen Monomeren copolymerisiert. Auch durch entsprechendes Nachaktivieren mit zusätzlichen Mengen an Radikalinitiatoren läßt sich der Monomerenumsatz kaum erhöhen bzw. der Restmonomerengehalt nur unwesentlich absenken. Hohe Anteile an Restmonomeren in Bindemitteln für Beschichtungszwecke sind naturgemäß gesundheitsschädlich, so daß die Bindemittel für diesen Anwendungszweck ungeeignet sind.

Die japanische Patentanmeldung 54 419 vom 31.03.1982 (C.A. 100, 211 268 (1984)) beschreibt Isocyanatgruppen aufweisendeCopolymerisate, die unter Mitverwendung von Umsetzungsprodukten von Hydroxyethylacrylat mit Diisocyanatotoluol bzw. mit Diisocyanatohexan hergestellt worden sind. Diese Copolymerisate sollen zur Herstellung von Klebstoffen verwendet werden. Ganz abgesehen von der Problematik der Herstellbarkeit von olefinisch ungesättigten Monomeren aus Hydroxyethylacrylat und Diisocyanaten mit Isocyanatgruppen gleicher oder annähernd gleicher Reaktivität (die Umsetzung der genannten Ausgangsmaterialien führt im allgemeinen stets zu großen Mengen an unerwünschten 2:1-Addukten), handelt es sich bei den in den Copolymerisaten dieser Vorveröffentlichung vorliegenden Isocyanatgruppen um solche einer vergleichsweise hohen Reaktivität, die zur Herstellung von Zweikomponenten-Lacken in Kombination mit stickstoffhaltigen Vernetzern wegen der unzureichenden Topfzeiten ungeeignet sind.

Grundsätzlich die gleichen Feststellungen rechtfertigen sich im Hinblick auf die Copolymerisate der japanischen Patentanmeldung 267 523 vom 20.12.1984 (C.A. 106, 157 585 (1987)), die ebenfalls zur Herstellung von Klebstoffen verwendet werden sollen, und bei deren Herstellung als Isocyanatgruppen aufweisende Monomere Umsetzungsprodukte von Isophorondiisocyanat mit Hydroxyethylacrylat verwendet werden.

Es war daher die der Erfindung zugrundeliegende Aufgabe, neue, olefinisch ungesättigte, als isocyanatfunktionelle Monomere geeignete Isocyanate, sowie diese als Bausteine enthaltende Copolymerisate zur Verfügung zu stellen, die nicht mit den Nachteilen des Standes der Technik behaftet sind.

Diese Aufgabe konnte durch die Bereitstellung der nachstehend näher beschriebenen olefinisch ungesättigten Monoisocyanate und der mit ihnen hergestellten Copolymerisate gelöst werden.

Gegenstand der Erfindung sind olefinisch ungesättigte Verbindungen des Molekulargewichtsbereichs 252 bis 800, gekennzeichnet durch

(i) einen Gehalt an Urethangruppen von 7 bis 24 Gew.-% und

(ii) einen Gehalt an Isocyanatgruppen von 5 bis 17 Gew.-% , die an tertiäre Kohlenstoffatome gebunden sind, die ihrerseits Teil eines cycloaliphatischen Rings sind.

wobei 1-Isocyanato-1-methyl-3-(2-allyloxy-ethoxycarbonylamino)-cyclohexan und 1-Isocyanato-1-methyl-3-(2-acryloxyethoxycarbonylamino)-cyclohexan, ausgenommen sind.

Gegenstand der erfindung ist auch ein Verfahren zur Herstellung von Isocyanatgruppen aufweisenden Copolymerisaten eines als Gewichtsmittel bestimmten Molekulargewichts von 1000 bis 100 000 durch radikalisch

initiierte Copolymerisation von isocyanatgruppenfreien, olefinisch ungesättigten Monomeren mit olefinisch ungesättigten Monoisocyanaten in einer solchen Menge, daß in den resultierenden Copolymerisaten 0,1 bis 13 Gew.-% an chemisch eingebauten Isocyanatgruppen bei einer mittleren NCO-Funktionalität der Copolymerisate von mindestens 2 vorliegen, dadurch gekennzeichnet, daß man als olefinisch ungesättigte Monoisocyanate ungesättigten Verbindungen der erfindungsgemäßen Art verwendet.

Gegenstand der erfindung sind schließlich auch Beschichtungs- oder Dichtmassen, die als Bindemittel ein Zweikomponenten-System aus einer Polyisocyanatkomponente und einer Polyaminkomponente mit blockierten Aminogruppen enthalten, dadurch gekennzeichnet, daß die Polyisocyanatkomponente aus nach diesem Verfahren hergestellten Copolymerisaten besteht.

Bei den erfindungsgemäßen, olefinisch ungesättigten Verbindungen handelt es sich um einfach olefinisch ungesättigte Monoisocyanate, die vorzugsweise ein Molekulargewicht von ca. 252 bis 800, d.h. einen NCO-Gehalt von 5 bis 17 Gew.-% und einen Gehalt an Urethangruppen von 7 bis 24 Gew.-%, aufweisen. Die Isocyanatgruppe ist in den erfindungsgemäßen Verbindungen an ein tertiäres Kohlenstoffatom gebunden, welches seinerseits Teil eines cycloaliphatischen Rings ist.

Die US-PS 2 958 704 beschreibt eine Reihe von olefinisch ungesättigten, Urethangruppen aufweisenden Isocyanaten, jedoch handelt es sich bei den Isocyanaten der Vorveröffentlichung mit einer Ausnahme um solche, deren Isocyanatgruppen nicht an ein tertiäres Kohlenstoffatom gebunden sind, welches Teil eines cycloaliphatischen Rings ist. Die Ausnahme bezieht sich auf eine Verbindung unklarer Konstitution. Aus der übrigen Offenbarung kann hergeleitet werden, daß die Autoren der Vorveröffentlichung eine der oben genannten Verbindungen gemeint haben könnten. Diese Verbindungen wurden jedoch per Disclaimer vom Patentschutz ausgenommen und sind nicht Gegenstand der vorliegenden Erfindung.

Die erfindungsgemäßen, olefinisch ungesättigten Verbindungen werden hergestellt durch Umsetzung von speziellen Ausgangsdiisocyanaten, welche eine aliphatisch gebundene primäre Isocyanatgruppe und eine cycloaliphatisch gebundene tertiäre Isocyanatgruppe aufweisen, mit hydroxylfunktionellen olefinisch ungesättigten Verbindungen in der Weise, daß pro Molekül Diisocyanat ein Molekül Hydroxylverbindung zum Einsatz kommt. Dabei können gegebenenfalls Polymerisationsinhibitoren wie z.B. 2,6-Di-tert.-butyl-4-methylphenol oder Hydrochinonmonomethylether zugesetzt werden.

Es handelt sich bei den Ausgangsdiisocyanaten um aliphatisch-cycloaliphatische Diisocyanate mit einem NCO-Gehalt von 20 bis 50, vorzugsweise 30 bis 48 Gew.-%, die neben einer sterisch ungehinderten, aliphatisch gebundenen Isocyanatgruppe eine sterisch gehinderte Isocyanatgruppe aufweisen, die an ein tertiäres Kohlenstoffatom gebunden ist, welches seinerseits Teil eines cycloaliphatischen Rings ist. Gut geeignete Ausgangsdiisocyanate sind solche der Formel (I) oder beliebige Gemische von solchen der Formel (I)

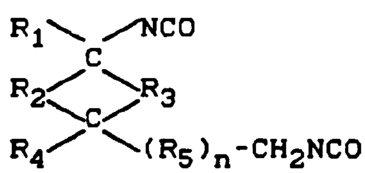

(I)

für welche

$R_1$ für einen Alkylrest mit 1 bis 4 Kohlenstoffatomen, vorzugsweise einen Methylrest steht,

$R_2$ und $R_3$ für gleiche oder verschiedene Reste stehen und jeweils einen zweiwertigen linearen oder verzweigten gesättigten Kohlenwasserstoffrest mit 1 bis 4, vorzugsweise 1 bis 3 Kohlenstoffatomen bedeuten, wobei die Summe der Kohlenstoffatome dieser Reste vorzugsweise 3 bis 6, insbesondere 4 oder 5 beträgt,

$R_4$ für Wasserstoff oder einen Alkylrest mit 1 bis 4 Kohlenstoffatomen, vorzugsweise für Wasserstoff oder einen Methylrest steht,

$R_5$ für einen zweiwertigen, linearen oder verzweigten, gesättigten aliphatischen Kohlenwasserstoffrest mit 1 bis 4, insbesondere 1 bis 3 Kohlenstoffatomen steht und

n für 0 oder 1 steht.

Besonders bevorzugte Diisocyanate sind z.B. 1-Isocyanato-1-methyl-4(3)-isocyanatomethylcyclohexan, welches im allgemeinen als Gemisch der 4- und 3-Isocyanatomethyl-Isomeren vorliegt, 1-Isocyanato-1-methyl-4-(4-isocyanatobut-2-yl)-cyclohexan oder 1-Isocyanato-1,2,2-trimethyl-3-(2-isocyanatoethyl)-cyclopentan. Geeignet sind jedoch auch z.B. 1-Isocyanato-1-n-butyl-3-(4-isocyanatobut-1-yl)-cyclopentan, 1-Isocyanato-1-ethyl-4-n-butyl-4-(4-isocyanatobut-1-yl)-cyclohexan oder 1-Isocyanato-1,2-dimethyl-3-ethyl-3-isocyanatomethylcyclopentan.

Die entsprechenden Diisocyanate sind in der EP-A-0 153 561 ausführlich beschrieben und können nach

den dort angegebenen Verfahren hergestellt werden.

Als hydroxyfunktionelle olefinisch ungesättigte Verbindung kommen beliebige organische Verbindungen in Betracht, die eine alkoholische Hydroxylgruppe und eine olefinische Doppelbindung aufweisen. Vorzugsweise weisen diese Verbindungen ein Molekulargewicht von 58 bis 600 auf. Geeignet sind beispielsweise Hydroxyethylacrylat, 2- und 3-Hydroxypropylacrylat, 2-, 3- und 4-Hydroxybutylacrylat, Hydroxyethylmethacrylat, 2- und 3-Hydroxypropylmethacrylat, Hydroxyethylvinylether, 2-, 3- und 4-Hydroxybutylvinylether, Allylalkohol, Umsetzungsprodukte von ungesättigten Carbonsäuren wie z.B. Acrylsäure mit Epoxiden, Umsetzungsprodukte der zuvor beispielhaft genannten hydroxyfunktionellen olefinisch ungesättigten Monomeren mit ε-Caprolacton, Butyrolacton, Ethylenoxid oder Propylenoxid.

Die Umsetzung der speziellen Diisocyanate mit den zuvor beispielhaft genannten hydroxylfunktionellen olefinisch ungesättigten Monomeren erfolgt zweckmäßigerweise so, daß je Mol Diisocyanat ein Mol Hydroxylverbindung zum Einsatz kommt.

Die Reaktion kann sowohl in An- als auch in Abwesenheit eines gegenüber Isocyanat- und Hydroxylgruppen inerten Lösungsmittels durchgeführt werden. Die Reaktionstemperatur liegt zwischen 0 und 100°C, vorzugsweise zwischen 20 und 80°C. Weiterhin können auch Katalysatoren wie z.B. tertiäre Amine, organische Zink- oder Zinnverbindungen oder andere, eine Urethanbildungsreaktion katalysierende Verbindungen in kleinen Mengen zugesetzt werden. Als Reaktionsprodukte werden in der Regel Monoaddukte aus den speziellen Ausgangsdiisocyanaten und den jeweils eingesetzten hydroxylfunktionellen olefinisch ungesättigten Verbindung erhalten, welche ohne zusätzliche Aufarbeitungs- oder Reinigungsprozesse ihrer Verwendung zur Herstellung der erfindungsgemäßen, isocyanatfunktionellen Copolymerisate zugeführt werden können.

Bei den Isocyanatgruppen-freien Monomeren, die mit den erfindungsgemäßen, olefinisch ungesättigten Monoisocyanaten beim erfindungsgemäßen Verfahren copolymerisiert werden, handelt es sich um die üblichen, vorzugsweise einfach olefinisch ungesättigten Monomeren, wie sie auch bereits bei dem Verfahren des obengenannten Standes der Technik als Monomere eingesetzt worden sind. Typische Beispiele sind Ester der Acryl- und Methacrylsäure wie beispielsweise Methylacrylat, Ethylacrylat, n-Butylacrylat, Isobutylacrylat, tert.-Butylacrylat, 2-Ethylhexylacrylat, Cyclohexylmethacrylat, Methylmethacrylat, n-Butylmethacrylat, Isobutylmethacrylat oder 2-Ethylhexylmethacrylat; Vinylaromaten wie beispielsweise Styrol, Vinyltoluol, α-Methylstyrol, α-Ethylstyrol, kernsubstituierte, gegebenenfalls Isomerengemische darstellende Diethylstyrole, Isopropylstyrole, Butylstyrole und Methoxystyrole, Vinylether wie beispielsweise Ethylvinylether, n-Propylvinylether, Isopropylvinylether, n-Butylvinylether und Isobutylvinylether; Vinylester wie beispielsweise Vinylacetat, Vinylpropionat und Vinylbutyrat.

Bei der Durchführung der Copolymerisation können selbstverständlich jeweils beliebige Gemische der beispielhaft genannten Monomeren verwendet werden.

Die Herstellung der Copolymerisate, d.h. die Durchführung des erfindungsgemäßen Verfahrens erfolgt durch Copolymerisation der beispielhaft genannten Monomeren, wobei die erfindungswesentlichen olefinisch ungesättigten Monoisocyanate in einer solchen Menge mitverwendet werden, daß in den resultierenden Copolymerisaten 0,1 bis 13 Gew.-%, vorzugsweise 1 bis 10 Gew.-% an freien Isocyanatgruppen vorliegen. In der Praxis bedeutet dies, daß im allgemeinen das Gewichtsverhältnis von Isocyanatgruppen aufweisenden Monomeren zu Isocyanatgruppen-freien Monomeren der beispielhaft genannten Art bei 0,01:1 bis 24:1 liegt. Die Copolymerisation erfolgt nach üblichen radikalischen Polymerisationsverfahren wie beispielsweise Masse- oder Lösungspolymerisation.

Dabei werden die Monomeren bei Temperaturen von 60 bis 200°C, vorzugsweise 80 bis 160°C in Gegenwart von Radikalbildnern und gegebenenfalls Molekulargewichtsreglern copolymerisiert.

Die Copolymerisation wird vorzugsweise in inerten Lösungsmitteln bei Feststoffgehalten von 30 bis 95 Gew.-% durchgeführt. Geeignete Lösungsmittel sind beispielsweise Aromaten wie Benzol, Toluol, Xylol, Ester wie Ethylacetat, Butylacetat, Methylglykolacetat, Ethylglykolacetat, Methoxypropylacetat, Ether wie Tetrahydrofuran, Dioxan, Diethylenglykoldimethylether, Ketone wie Aceton, Methylethylketon, Methylisobutylketon.

Die Copolymerisation kann kontinuierlich oder diskontinuierlich erfolgen. Üblicherweise wird in einen Polymerisationsreaktor gleichmäßig und kontinuierlich die Monomermischung und der Initiator eindosiert und gleichzeitig die entsprechende Menge Polymerisat kontinuierlich abgeführt. Vorzugsweise können so chemisch nahezu einheitliche Copolymere hergestellt werden. Chemisch nahezu einheitliche Copolymere können auch hergestellt werden, indem man die Reaktionsmischung mit konstanter Geschwindigkeit in einen Rührkessel einlaufen läßt, ohne das Polymerisat abzuführen.

Man kann auch einen Teil der Monomeren beispielsweise in Lösungsmitteln der genannten Art vorlegen und die restlichen Monomeren und Hilfsmittel getrennt oder gemeinsam in diese Vorlage bei der Reaktionstemperatur eintragen.

Im allgemeinen erfolgt die Polymerisation bei einem Überdruck von 0 bis 20 bar. Die Initiatoren werden in Mengen von 0,05 bis 15 Gew.-%, bezogen auf die Gesamtmenge der Monomeren, eingesetzt.

Geeignete Initiatoren sind übliche Radikalstarter wie z.B. aliphatische Azoverbindungen wie Azodiisobuttersäurenitril, Azo-bis-2-methylvaleronitril, 1,1'-Azo-bis-1-cyclohexannitril und 2,2'-Azo-bis-isobuttersäurealkylester; symmetrische Diacylperoxide, z.B. Acetyl-, Propionyl- oder Butyrylperoxid, mit Brom-, Nitro-, Methyl- oder Methoxygruppen substituierte Benzoylperoxide, Laurylperoxide; symmetrische Peroxidicarbonate, z.B. Diethyl-, Diisopropyl-, Dicyclohexyl- sowie Dibenzoylperoxidicarbonat; tert.-Butylperoctoat, tert.-Butylperbenzoat oder tert.-Butylphenylperacetat sowie Peroxycarbonate wie z.B. tert.-Butyl-N-(phenylperoxy)-carbonat oder tert.-Butyl-N-(2-, 3- oder 4-chlorphenylperoxy)-carbonat; Hydroperoxide wie beispielsweise tert.-Butylhydroperoxid, Cumolhydroperoxid; Dialkylperoxide wie Di-cumylperoxid; tert.-Butyl-cumylperoxid oder Di-tert.-butylperoxid.

Zur Regelung des Molekulargewichtes der Copolymerisate können übliche Regler bei der Herstellung eingesetzt werden. Beispielhaft genannt seien tert.-Dodecylmercaptan, n-Dodecylmercaptan oder Diisopropylxanthogendisulfid. Die Regler können in Mengen von 0,1 bis 10 Gew.-%, bezogen auf die Gesamtmenge der Monomeren, zugegeben werden.

Die Copolymerisate weisen im übrigen ein, nach der Methode der Gelpermeationschromatographie bestimmbares, Molekulargewicht (Gewichtsmittel) von 1000 bis 100 000, vorzugsweise 1500 bis 75 000 und besonders bevorzugt 2000 bis 50 000 auf.

Die bei der Copolymerisationsreaktion anfallenden Lösungen der Copolymerisate können ohne weitere Aufarbeitung der erfindungsgemäßen Verwendung zugeführt werden. Selbstverständlich ist es jedoch auch möglich, die Copolymerisate destillativ von gegebenenfalls noch vorliegenden Restmengen an nicht umgesetzten Monomeren und Lösungsmittel zu befreien und die als Destillationsrückstand vorliegenden Copolymerisate der erfindungsgemäßen Verwendung zuzuführen.

In den erfindungsgemäßen Beschichtungs- oder Dichtmassen liegen als Bindemittel Zweikomponenten-Systeme aus einer Polyisocyanatkomponente A) und einem Härter B) vor. Bei der Polyisocyanatkomponente A) handelt es sich um die erfindungsgemäßen isocyanatfunktionellen Copolymerisate. Bei der Härterkomponente B) handelt es sich um organische Polyamine mit blockierten Aminogruppen. Unter "blockierten Aminogruppen" sind dabei solche Gruppen zu verstehen, die mit Wasser unter Freisetzung von primären und/oder sekundären Aminogruppen reagieren. Besonders bevorzugte blockierte Polyamine B) sind Aldimin-, Ketimin-, Oxazolan-, Hexahydropyrimidin- und/oder Tetrahydroimidazolgruppen aufweisende Verbindungen, die gleichzeitig auch mehrere dieser Gruppierungen enthalten können.

Die blockierten Polyamine B) weisen ein als Gewichtsmittel bestimmtes Molekulargewicht von 86 bis 10 000, bevorzugt von 250 bis 4000, auf und enthalten im statistischen Mittel 1 bis 50, bevorzugt 2 bis 10, und insbesondere 2 bis 4, Struktureinheiten der allgemeinen Formel

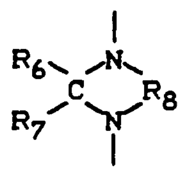 ,

(II)

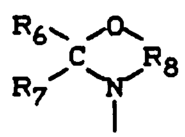

(III)

und/oder

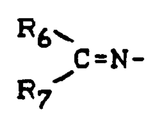

(IV)

wobei

$R_6$ und $R_7$ für gleiche oder verschiedene Reste stehen und Wasserstoff, aliphatische Kohlenwasserstoffreste mit 1 bis 18 Kohlenstoffatomen, cycloaliphatische Kohlenwasserstoffreste mit 5 bis 10 Kohlenstoffatomen, aliphatische Kohlenwasserstoffreste mit 7 bis 18 Kohlenstoffatomen oder Phenylreste bedeuten, wobei die beiden Reste $R_6$ und $R_7$ zusammen mit dem benachbarten Kohlenstoffatomen auch einen 5- oder 6-gliedrigen cycloaliphatischen Ring bilden können, und wobei vorzugsweise höchstens einer der Reste für Wasserstoff steht, und

$R_6$ für einen zweiwertigen aliphatischen Kohlenwasserstoffrest mit 2 bis 6 Kohlenstoffatomen steht, mit der Maßgabe, daß zwischen den beiden Stickstoffatomen 2 oder 3 Kohlenstoffe angeordnet sind.

Die genannten Molekulargewichte können nach der Methode der Gelpermeationschromatographie (Molekulargewichte von oberhalb 1000) bzw. aus der Stöchiometrie der zur Herstellung der Verbindungen eingesetzten Ausgangsmaterialien (Molekulargewichte bis 1000) ermittelt werden.

Bevorzugt eingesetzte Komponenten B) sind z.B. solche, die Hexahydropyrimidin- bzw. Tetrahydroimidazolstrukturen der allgemeinen Formel (II) enthalten, für welche $R_6$ und $R_7$ für gleiche oder verschiedene aliphatische Kohlenwasserstoffreste mit 2 bis 10 Kohlenstoffatomen stehen, wobei einer der Reste auch für Wasserstoff stehen kann und $R_7$ für einen gegebenenfalls alkylsubstituierten Ethylen- oder Trimethylenrest steht.

Die Herstellung von blockierten Polyaminen der genannten Art gelingen in an sich bekannter Weise durch Umsetzung von entsprechenden Aldehyden bzw. Ketonen mit den entsprechenden Polyaminen.

Zur Herstellung der Hexahydropyrimidin- bzw. Tetrahydroimidazolgruppen aufweisenden Verbindungen B) geeignete Aldehyde bzw. Ketone sind beispielsweise solche der allgemeinen Formel

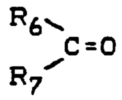 ,

die vorzugsweise ein Molekulargewicht von 72 bis 200 (Ketone) bzw. von 58 bis 250 (Aldehyde) aufweisen.

Beispiele hierfür sind Aceton, Methylethylketon, Methylpropylketon, Methylisopropylketon, Methyl-n-butylketon, Methylisobutylketon, Methyl-n-amylketon, Methylisoamylketon, Methylheptylketon, Diethylketon, Ethylbutylketon, Ethylamylketon, Diisopropylketon, Diisobutylketon, Cyclohexanon, Isophoron, Methyl-tert.-butylketon, 5-Methyl-3-heptanon, 4-Heptylketon, 1-Phenyl-2-propanon, Acetophenon, Methylnonylketon, 3,3,5-Trimethylcyclohexanon, Formaldehyd, Acetaldehyd, Propionaldehyd, Butyraldehyd, Isobutyraldehyd, Trimethylacetaldehyd, 2,2-Dimethylpropanol, 2-Ethylhexanol, 3-Cyclohexen-1-carboxaldehyd, Hexanal, Heptanal, Octanal, Valeraldehyd, Benzaldehyd, Tetrahydrobenzaldehyd, Hexahydrobenzaldehyd, Acrolein, Crotonaldehyd, Propargylaldehyd, p-Toluylaldehyd, 2-Methylpentanal, 3-Methylpentanal, Phenylethanal, 4-Methylpentanal.

Zur Herstellung der Hexahydropyrimidin- bzw. Tetrahydroimidazolgruppen aufweisenden Verbindungen bevorzugte Aldehyde und Ketone sind Butyraldehyd, Isobutyraldehyd, Trimethylacetaldehyd, 2,2-Dimethylpropanol, 2-Ethylhexanal, Hexanal, 3-Cyclohexan-1-carboxaldehyd, Heptanal, Octanal, Hexahydrobenzaldehyd, 2-Methylpentanal, Cyclohexanon, Cyclopentanon, Methylisopropylketon, Aceton, 3,3,5-Trimethylcyclohexanon und Methylcyclohexanon.

Es können selbstverständlich auch Mischungen verschiedener Ketone bzw. Aldehyde und auch Mischungen von Ketonen mit Aldehyden eingesetzt werden, um spezielle Eigenschaften zu erzielen.

Bei den zur Herstellung der Hexahydropyrimidin- bzw. Tetrahydroimidazolgruppen aufweisenden Verbindungen zum Einsatz gelangenden Polyaminen handelt es sich insbesondere um organische Verbindungen, die mindestens zwei primäre und/oder sekundäre Aminogruppe aufweisen.

Geeignete Polyamine sind beispielsweise solche der allgemeinen Formel

$$R_9\text{-NH-}R_8\text{-NH-}R_{10},$$

in welcher

$R_8$ die obengenannte Bedeutung hat und

$R_9$ und $R_{10}$ für gleiche oder verschiedene Reste stehen und Wasserstoff, aliphatische Kohlenwasserstoffreste mit 1 bis 10, bevorzugt 1 bis 4 Kohlenstoffatomen, cycloaliphatische Kohlenwasserstoffreste mit 5 bis 10, vorzugsweise 6 Kohlenwasserstoffatomen oder aromatische Kohlenwasserstoffreste mit 7 bis 15, vorzugsweise 7 Kohlenwasserstoffatomen, stehen, wobei die genannten Kohlenwasserstoffreste, insbesondere die genannten aliphatischen Kohlenwasserstoffreste, gegebenenfalls Heteroatome wie Sauerstoff, Stickstoff oder Schwefel in Form von Ether-, Ester-, Amid-, Urethan-, Oxiran-, Keton-, Lactam-, Harnstoff-, Thioether-, Thioester-oder Lactongruppen aufweisen können, oder wobei die Reste auch reaktionsfähige Hydroxyl-oder Aminogruppen aufweisen können.

Besonders bevorzugte Polyamine sind solche, bei denen $R_9$ und $R_{10}$ für gleiche oder verschiedene Reste stehen und Wasserstoff und/oder $C_1$-$C_6$-Alkylreste wie Methyl-, Ethyl-, n-Propyl-, iso-Propyl-, n-Butyl-, iso-Butyl-, tert.-Butyl-, n-Pentyl- oder n-Hexylreste bedeuten, oder wobei zumindest einer der Reste $R_8$ bzw. $R_9$ für einen Rest steht, wie er durch Anlagerung eines Aminwasserstoffatoms an eine olefinisch ungesättigte Verbindung erhalten wird. Zur Herstellung derartiger, modifizierter Polyamine geeignete, olefinisch ungesättigte Verbindungen sind beispielsweise Derivate der (Meth)Acrylsäure wie Ester, Amide, Nitrile oder z.B. Vinylaromaten wie Styrol, $\alpha$-Methylstyrol, Vinyltoluol oder z.B. Vinylester wie Vinylacetat, Vinylpropionat, Vinylbutyrat oder z.B. Vinylether wie Ethylvinylether, Polyvinylether, Butylvinylether oder Mono- und Diester der Fumarsäure, Maleinsäure oder Tetrahydrophthalsäure.

$R_9$ und/oder $R_{10}$ können auch für einen Aminoalkyl- oder Hydroxyalkylrest mit beispielsweise 2 bis 4 Kohlenstoffatomen stehen.

Ganz besonders bevorzugte Polyamine sind Ethylendiamin, 1,2-Propylendiamin, 1,3-Propylendiamin, 1,2- und 1,3-Butylendiamin, Diethylentriamin sowie Derivate dieser Polyamine.

Als Komponente B) geeignete, Oxazolangruppen der allgemeinen Formel (III) aufweisende Verbindungen sind vorzugsweise solche, für welche $R_6$ und $R_7$ für gleiche oder verschiedene Reste stehen und Wasserstoff oder aliphatische Kohlenwasserstoffreste mit 1 bis 18 und insbesondere 1 bis 8 Kohlenstoffatomen stehen,

oder wobei die Reste $R_6$ und $R_7$ zusammen mit dem Kohlenstoffatom des heterocyclischen Rings einen cycloaliphatischen Ring mit insgesamt 4 bis 9 Kohlenstoffatomen, insbesondere einen Cyclohexanring bilden, mit der Maßgabe, daß höchstens einer der Reste $R_6$ und $R_7$ für Wasserstoff steht, und wobei $R_8$ für einen Alkylenrest mit 2 bis 4, vorzugsweise 2 bis 3 Kohlenstoffatomen steht, mit der Maßgabe, daß zwischen dem Sauerstoff- und dem Stickstoffatom mindestens 2 Kohlenstoffatome angeordnet sind.

Die Herstellung der oxazolanhaltigen Komponente B) gelingt in an sich bekannter Weise durch Umsetzung von entsprechenden Aldehyden bzw. Ketonen der Formel

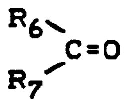

mit geeigneten Hydroxyaminen der nachstehend näher genannten Art.

Als Aldehyde bzw. Ketone kommen grundsätzlich solche der oben bereits beispielhaft genannten Art in Betracht. Bevorzugte Aldehyde bzw. Ketone sind hier Butyraldehyd, Isobutyraldehyd, Trimethylacetaldehyd, 2,2-Dimethylpropanal, 2-Ethylhexanal, 3-Cyclohexen-1-carboxaldehyd, Hexahydrobenzaldehyd, Cyclopentanon, Cyclohexanon, Methylcyclopentanon, Methylcyclohexanon, 3,3,5-Trimethylcyclohexanon, Cyclobutanon, Methylcyclobutanon, Aceton, Methylethylketon und Methylisobutylketon.

Es können selbstverständlich auch Mischungen verschiedener Ketone bzw. Aldehyde und auch Mischungen von Ketonen mit Aldehyden eingesetzt werden, um spezielle Eigenschaften zu erzielen.

Bei Hydroxyaminen handelt es sich insbesondere um organische Verbindungen, die mindestens eine aliphatische Aminogruppe und mindestens eine aliphatisch gebundene Hydroxylgruppe aufweisen. Die Hydroxyamine weisen im allgemeinen ein zwischen 61 und 500, vorzugsweise zwischen 61 und 300 liegendes Molekulargewicht auf.

Geeignete Hydroxyamine können z.B. sein: Bis-(2-hydroxyethyl)-amin, Bis-(2-hydroxypropyl)-amin, Bis-(2-hydroxybutyl)-amin, Bis-(3-hydroxypropyl)-amin, Bis-(3-hydroxyhexyl)-amin, N-(2-hydroxypropyl)-N-(2-hydroxyethyl)-amin, 2-(Methylamino)-ethanol, 2-(Ethylamino)-ethanol, 2-(Propylamino)-ethanol, 2-(Butylamino)-ethanol, 2-Amino-2-methyl-1-propanol, 2-Amino-2-ethyl-1-propanol, 2-Amino-2-propyl-1-propanol, 2-Amino-2-methyl-1,3-propandiol, 2-Amino-3-methyl-3-hydroxybutan, Propanolamin, Ethanolamin.

Ganz besonders bevorzugt sind: Bis-(2-hydroxyethyl)-amin, Bis-(2-hydroxypropyl)-amin, Bis-(2-hydroxybutyl)-amin, Bis-(3-hydroxyhexyl)-amin, 2-(Methylamino)-ethanol, 2-(Ethylamino)-ethanol, 2-Amino-2-methyl-1-propanol, 2-Amino-2-ethyl-1-propanol, Propanolamin und Ethanolamin.

Zu den bevorzugten Aldimin- bzw. Ketimingruppen aufweisenden Verbindungen gehören solche, die Struktureinheiten der allgemeinen Formel (IV)

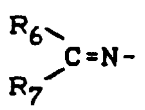 (IV)

enthalten, wobei

$R_6$ und $R_7$ für gleiche oder verschiedene Reste stehen und Wasserstoff oder aliphatische Kohlenwasserstoffreste mit 1 bis 8 Kohlenstoffatomen darstellen, die auch zusammen mit dem Kohlenstoffatom zu einem cycloaliphatischen Ring, insbesondere einem Cyclohexanring verknüpft sein können.

Zur Herstellung dieser Verbindungen sind im Prinzip die bereits oben beispielhaft genannten Aldehyde bzw. Ketone geeignet. Bevorzugte Aldehyde bzw. Ketone sind hier Butyraldehyd, Isobutyraldehyd, Trimetylacetaldehyd, 2,2-Dimethylpropanal, 2-Ethylhexanal, 3-Cyclohexen-1-carboxaldehyd, Hexahydrobenzaldehyd und insbesondere solche Ketone, die einen Siedepunkt von unter 170°C aufweisen und bei Raumtemperatur eine gute Flüchtigkeit zeigen, wie z.B. Methylisobutylketon, Methylisopropylketon, Diethylketon, Diisobutylketon, Methyl-tert.-butylketon.

Es können selbstverständlich auch Mischungen verschiedener Ketone bzw. Aldehyde und auch Mischungen von Ketonen mit Aldehyden eingesetzt werden, um spezielle Eigenschaften zu erzielen.

Bei den zur Herstellung von Ketimin- bzw. Aldimingruppen enthaltenden Komponente B) zum Einsatz gelangenden Polyaminen handelt es sich insbesondere um organische Verbindungen, die mindestens zwei aliphatisch und/oder cycloaliphatisch gebundene primäre Aminogruppen aufweisen. Die Verwendung von solchen Polyaminen, die aromatische Aminogruppen aufweisen, ist zwar ebenfalls möglich, jedoch weniger bevorzugt. Die Polyamine weisen im allgemeinen ein von 60 bis 500, vorzugsweise von 88 bis 400 liegendes

Molekulargewicht auf, jedoch können auch höhermolekulare, endständige Aminogruppen aufweisende Prepolymere als Polyaminkomponente bei der Herstellung der Komponente B) eingesetzt werden.

Besonders bevorzugte Polyamine sind diprimäre aliphatische bzw. cycloaliphatische Diamine wie beispielsweise Tetramethylendiamin, Hexamethylendiamin, Isophorondiamin, Bis-(4-aminocyclohexyl)-methan, Bis-aminomethylhexahydro-4,7-methanoindan, 1,4-Cyclohexandiamin, 1,3-Cyclohexandiamin, 2-Methylcyclohexandiamin, 4-Methylcyclohexandiamin, 2,2,5-Trimethylhexandiamin, 2,2,4-Trimethylhexandiamin, 1,4-Butandiol-bis-(3-aminopropyl)-ether, 2,5-Diamino-2,5-dimethylhexan, Bis-amino-methylcyclohexan, Bis-(4-amino-3,5-dimethylcyclohexyl)-methan oder deren Gemische.

Ganz besonders bevorzugt sind Tetramethylendiamin, Hexamethylendiamin, Isophorondiamin, Bis-aminomethylcyclohexan, 1,4-Cyclohexandiamin, Bis-aminomethylhexahydro-4,7-methanoindan und Bis-(4-aminocyclohexyl)-methan.

Neben diesen bevorzugten Diaminen können zur Herstellung der Aldimine bzw. Ketimine auch endständige primäre Aminogruppen aufweisende Prepolymere eingesetzt werden, d.h. Verbindungen, die mindestens zwei endständige Aminogruppen und ein Molekulargewicht von 500 bis 5000, vorzugsweise 500 bis 2000, aufweisen. Zu diesen Verbindungen gehören beispielsweise die aus der Polyurethanchemie an sich bekannten Aminopolyether, wie sie beispielsweise in der EP-A 0 081 701 beschrieben werden, oder z.B. Amid-, Harnstoff-,

Urethan- oder sekundäre Aminogruppen aufweisende Umsetzungsprodukte von mindestens difunktionellen Carbonsäuren, Isocyanaten oder Epoxiden mit Diaminen der oben beispielhaft genannten Art, wobei diese Umsetzungsprodukte noch mindestens zwei primäre Aminogruppen aufweisen. Auch Gemische von derartigen höhermolekularen Polyaminen mit den beispielhaft genannten niedermolekularen Polyaminen können zum Einsatz gelangen.

Zu den aromatischen Polyaminen, die im Prinzip auch zur Herstellung der Aldimine bzw. Ketimine geeignet, jedoch weniger bevorzugt sind, gehören beispielsweise 2,4- und 2,6-Diaminotoluol, 1,4-Diaminobenzol oder 4,4′-Diaminodiphenylmethan.

Die Herstellung der Komponente B), welche Aldimin, Ketimin-, Oxazolan-, Hexahydropyrimidin- oder Tetrahydroimidazolgruppen enthalten kann, erfolgt durch Umsetzung der Ausgangskomponenten, wobei im allgemeinen die Mengenverhältnisse der Reaktionspartner so gewählt werden, daß die Aminoverbindungen, bezogen auf die jeweilig angestrebte Reaktion, in 1- bis 1,5-fachem molarem Überschuß, bezogen auf die Carbonylgruppen, vorliegen. Gegebenenfalls können zur Reaktionsbeschleunigung katalytische Mengen von sauren Substanzen wie z.B. p-Toluolsulfonsäure, Salzsäure, Schwefelsäure, Aluminium-III-chlorid, Zinnverbindungen eingesetzt werden.

Die Umsetzung erfolgt im allgemeinen innerhalb des Temperaturbereichs von 60 bis 180°C, wobei die Reaktion in Gegenwart eines Schleppmittels zur Entfernung des Reaktionswassers so lange durchgeführt wird, bis die berechnete Menge an Wasser abgespalten ist bzw. bis kein Wasser mehr abgespalten wird.

Anschließend werden Schleppmittel und gegebenenfalls vorliegende, nicht umgesetzte Ausgangsmaterialien destillativ abgetrennt. Geeignete Schleppmittel sind z.B. Toluol, Xylol, Cyclohexan, Octan. Die so erhaltenen Rohprodukte können ohne weitere Reinigungsschritte als Komponente B) zur Herstellung der Bindemittelkombinationen verwendet werden. Werden besonders hohe Anforderungen an die Reinheit der Komponente B) gestellt, so ist es auch möglich, z.B. durch eine Destillation, die Komponenten B) in reiner Form zu erhalten.

Zu den bevorzugten, blockierten Polyaminen der Komponente B) gehören auch solche, in denen 2 bis 10 Struktureinheiten der allgemeinen Formel (II), (III), (IV) enthalten sind, und die durch Verknüpfung von unterschiedlichen, derartige Struktureinheiten aufweisenden Verbindungen unter Ausbildung von z.B. Ester-, Ether-, Amid-, Harnstoff- und/oder Urethanbindungen erhalten werden.

Die auf diese Weise miteinander zu verknüpfenden, Struktureinheiten der genannten Formeln enthaltenden Verbindungen müssen mindestens eine primäre bzw. sekundäre Aminogruppe oder mindestens eine Hydroxylgruppe in nicht blockierter Form enthalten. Geeignete Modifizierungsmittel, die zur Verknüpfung der genannten Verbindungen geeignet sind, sind z.B. Polyisocyanate, Polyepoxide, Polycarbonsäuren und Polyacryloylverbindungen.

Für diese Modifizierungsreaktion geeignete Polyisocyanate sind beispielseise aliphatische, cycloaliphatische, aliphatische, aromatische und heterocyclische Polyisocyanate, wie sie z.B. von W.Siefken in Justus Liebigs Annalen der Chemie, 562, Seite 75 bis 1346, beschrieben werden, beispielsweise 1,4-Tetramethylendiisocyanat, 1,6-Hexamethylendiisocyanat, 1,12-Dodecandiisocyanat, Cyclobutan-1,3-diisocyanat, Cyclohexan-1,3-diisocyanat, Cyclohexan-1,4-diisocyanat, 1-Isocyanato-3,3,5-trimethyl-5-isocyanatomethyl-cyclohexan, 2,4- und 2,6-Hexahydrotoluylendiisocyanat, Hexahydro-1,3- und -1,4-phenylendiisocyanat, Perhydro-2,4′-und/oder -4,4′-diphenylmethandiisocyanat, 1,3-Phenylendiisocyanat, 1,4-Phenylendiisocyanat, 2,4-Toluylendiisocyanat, 2,6-Toluylendiisocyanat, Diphenylmethan-2,4′-und/oder -4,4′-diisocyanat, Naphthylen-1,5-diisocyanat, Gemische dieser und anderer Polyisocyanate, Polyisocyanate mit Carbodiimidgruppen (z.B. DE-OS 10 92 007), Polyisocyanate mit Allophanatgruppen (z.B. GB-PS 944 890), Isocyanuratgruppen aufweisende Polyisocyanate (z.B. DE-PS 10 22 789, DE-PS 12 22 067), Urethangruppen aufweisende Polyisocyanate (z.B. US-PS 33 94 164) oder durch Umsetzung von mindestens difunktionellen Hydroxyverbindungen mit überschüssigen, mindestens difunktionellen Isocyanaten hergestellte Polyisocyanate, Biuretgruppen aufweisende Polyisocyanate (z.B. DE-PS 11 01 394) und präpolymere bzw. polymere Substanzen mit mindestens zwei Isocyanatgruppen.

Vertreter dieser erfindungsgemäß zu verwendenden Verbindungen sind z.B. in High Polymers, Vol. XVI, "Polyurethans, Chemistry and Technology" Interscience Publishers, New York, London, Band I, 1962, S. 32 bis 42 bzw. 45 bis 54 und Band II, 1964, S. 5 bis 6 und 198 bis 199, sowie im Kunststoffhandbuch, Band VII, Vieweg-Höchtlen, Carl-Hanser-Verlag, München, 1966, S. 45 bis 72, beschrieben.

Für die genannte Modifizierungsreaktion geeignete Polyepoxide sind beispielsweise aliphatische, cycloaliphatische, aliphatische, aromatische und heterocyclische, mindestens zwei Epoxidgruppen enthaltende Substanzen, wie z.B. epoxidierte Ester aliphatischer polybasischer Säuren mit ungesättigten einwertigen Alkoholen, Glycidylether von Polyhydroxyverbindungen, Glycidylester von Polycarbonsäuren, epoxidgruppenhaltige Copolymerisate.

Für die Modifizierungseaktion geeignete Polycarbonsäuren sind beispielsweise aliphatische, cycloaliphatische, aliphatische, aromatische und heterocyclische, mindestens zwei Carboxylgruppen enthaltende Substanzen, wie z.B. Adipinsäure, Dimerfettsäure, Phthalsäure, Terephthalsäure, Isophthalsäure, Fumarsäure, Maleinsäure, Bernsteinsäure, Trimellithsäure, Pyromellithsäure, (Meth)acrylsäure-haltige Copolymerisate, saure Polyester oder saure Polyamide.

Anstelle der beispielhaft genannten Säuren können auch die entsprechenden Säureanhydride (sofern es sich um intramolekulare Säureanhydride bildende Säuren handelt) oder die entsprechenden einfachen Alkylester, insbesondere Methylester für Modifizierungsreaktion eingesetzt werden.

Für die Modifizierungsreaktion geeignete Verbindungen mit mindestens zwei olefinischen Doppelbindungen der genannten Art sind insbesondere Derivate der Acrylsäure bzw. Methacrylsäure wie beispielsweise Hexandiol-bis-(meth)acrylsäureester, Trimethylolpropan-tris-(meth)acrylsäureester, Pentaerythrittetra(meth) acrylsäureester, OH-funktionelle Polyester bzw. Polyacrylate, die mit Acrylsäure verestert sind, Diethylenglykoldimethacrylsäureester, Umsetzungsprodukte von Polyisocyanaten mit Hydroxyalkyl(meth)acrylat.

Bei der Modifizierungsreaktion zur Herstellung von höherfunktionellen Komponenten B) ist es auch möglich, Gemische verschiedener blockierter Amine einzusetzen, die jeweils mindestens eine freie, mit dem Modifizierungsmittel reaktionsfähige Hydroxyl- oder Aminogruppe aufweisen.

Ketimin- bzw. Aldimingruppen-haltige Polyamine, die noch mindestens eine freie primäre bzw. sekundäre Aminogruppe oder eine freie Hydroxylgruppe enthalten, erhält man z.B. durch Umsetzung von mindestens difunktionellen Aminen mit Ketonen und/oder Aldehyden in solchen Äquivalentverhältnissen, daß mindestens eine Aminogruppe frei bleibt.

Auch bei der Verwendung von beispielsweise solchen Polyaminen, die neben primären Aminogruppen mindestens eine sekundäre Aminogruppe aufweisen, entstehen bei der Umsetzung mit Aldehyden oder Ketonen Aldimine bzw. Ketimine, die mindestens eine freie sekundäre Aminogruppe aufweisen (falls unter Einhaltung eines Äquivalentverhältnisses von primären Aminogruppen zu Carbonylgruppen von 1:1 gearbeitet worden ist), bzw. die neben mindestens einer sekundären Aminogruppe noch freie primäre Aminogruppen enthalten (falls die Carbonylverbindungen, bezogen auf die primäre Aminogruppen, im Unterschuß verwendet worden sind). Derartige primär-sekundäre Polyamine sind beispielsweise Diethylentriamin, Triethylentetramin, Tetraethylenpentamin, Dipropylentriamin, Tripropylentetramin.

Oxazolangruppen enthaltende Verbindungen, die noch mindestens eine reaktive primäre bzw. sekundäre Aminogruppe oder eine Hydroxygruppe aufweisen, erhält man z.B. durch Umsetzung von Hydroxyaminen, die neben einer Hydroxygruppe und einer sekundären Aminogruppe noch mindestens eine weitere Hydroxygruppe und/oder primäre bzw. sekundäre Aminogruppe aufweisen, oder durch Umsetzung von geeigneten Hydroxyaminen mit einer Hydroxygruppe und einer primären Aminogruppe in geeigneten Äquivalentverhältnissen mit Ketonen und/oder Aldehyden, wie sie z.B. weiter oben beschrieben worden sind. Geeignete Hydroxyamine können z.B. sein: Bis-(2-hydroxyethyl)-amin, Bis-(2-hydroxypropyl)-amin, Bis-(2-hydroxybutyl)-amin, Bis-(3-hydroxypropyl)-amin, Bis-(3-hydroxyhexyl)-amin, N-(2-hydropropyl)-N-(6-hydroxyhexyl)-amin, 2-Amino-2-methyl-1-propanol, 2-Amino-2-methyl-1,3-propandiol, 2-Amino-3-methyl-3-hydroxybutan, Aminoethanol.

Die Herstellung der Oxazolangruppen enthaltenden Hydroxyamine, die noch mindestens eine freie primäre bzw. sekundäre Aminogruppe bzw. eine Hydroxygruppe aufweisen, erfolgt durch Umsetzung der genannten Ausgangskomponenten unter Einhaltung eines solchen Äquivalentverhältnisses von Amino- bzw. Hydroxygruppen zu Aldehyd- bzw. Ketongruppen, daß mindestens eine primäre bzw. sekundäre Aminogruppe bzw. eine Hydroxygruppe nicht blockiert wird und für die anschließende Umsetzung mit dem als Modifizierungsmittel verwendeten Reaktionspartner zur Verfügung steht.

Hexahydropyrimidin- bzw. Tetrahydroimidazolgruppen enthaltende Verbindungen, die noch mindestens eine reaktive primäre bzw. sekundäre Aminogruppe oder eine Hydroxygruppe aufweisen, erhält man z.B. durch Umsetzung von Hydroxyaminen, die neben mindestens einer Hydroxygruppe noch zwei sekundäre Aminogruppen enthalten, wie z.B. N-Methyl-N'-4-hydroxytetramethylendiamin, oder durch Umsetzung von Polyaminen, die neben mindestens einer sekundären Aminogruppe mindestens eine primäre Aminogruppe oder mindestens zwei weitere sekundäre Aminogruppen enthalten, wie z.B. N-Methyl-1,3-diaminoethan, N-Methyl-1,3-diaminopropan, N-Methyl-1,3-diaminobutan, Diethylentriamin, N-Methyldiethylentriamin, N,N-Bis-(3-aminopropyl)-amin, N,N'-Dimethyl-diethylentriamin.

Die Herstellung der Hexahydropyrimidin- bzw. Tetrahydroimidazolgruppen enthaltenden Verbindungen, welche noch mindestens eine freie primäre bzw. sekundäre Aminogruppe bzw. eine Hydroxylgruppe aufweisen, erfolgt durch Umsetzung der genannten Ausgangsverbindungen unter Einhaltung eines solchen Äquivalentverhältnisses von Amino-bzw. Hydroxygruppen zu Aldehyd- bzw. Ketogruppen, daß mindestens eine primäre bzw. sekundäre Aminogruppe bzw. eine Hydroxylgruppe nicht blockiert wird und für die anschließende Umsetzung mit dem als Modifizierungsmittel verwendeten Reaktionspartner zur Verfügung steht.

Zur näheren Erläuterung seien folgende Struktureinheiten, welche zum Aufbau solcher höhermolekularer, Ester-, Ether-, Amid-, Harnstoff-, Urethanbindungen enthaltender Komponenten B) geeignet sind, beispielhaft genannt:

- Bisketimin aus Diethylentriamin und Aceton

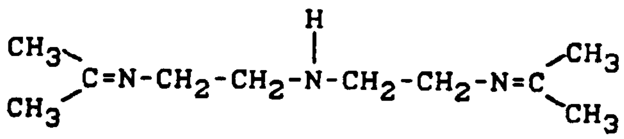

- Aldimin aus Isophorondiamin und Isobutyraldehyd

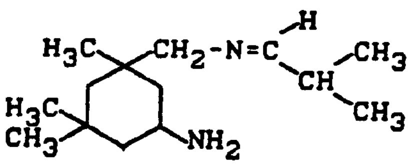

- Oxazolan aus Diethanolamin und Isobutyraldehyd

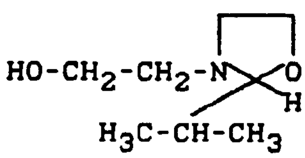

- Hexahydropyrimidin aus N-Methyl-1,3-diaminopropan und Cyclohexanon

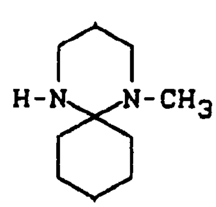

Falls die oben beispielhaft genannten Modifizierungsmittel mit freien primären bzw. sekundären Amino- bzw. Hydroxylgruppen aufweisenden Hexahydropyrimidinen bzw. Tetrahydroimidazolen umgesetzt werden, entstehen höherfunktionelle Hexahydropyrimidine bzw. Tetrahydroimidazole. Entsprechendes gilt für die Modifizierung von Aldiminen bzw. Ketiminen und für die Modifizierung von Oxazolanen.

Falls die Modifizierungsmittel jedoch mit Gemischen aus Hexahydropyrimidinen, Tetrahydroimidazolen, Aldiminen, Ketiminen und/oder Oxazolanen, welche freie primäre bzw. sekundäre Amino- bzw. Hydroxylgruppen aufweisen, umgesetzt werden, entstehen Vernetzungskomponenten B), in denen Hexahydropyrimidine, Tetrahydroimidazole, Ketimine, Aldimine und/oder Oxazolane chemisch miteinander verknüpft sind.

Durch diese Modifizierungs- bzw. Verknüpfungsreaktionen sind somit die unterschiedlichsten, als Komponente B) geeigneten Verbindungen zugänglich.

Die Modifizierungsreaktion wird üblicherweise in einem Lösungsmittel der oben beispielhaft genannten Art bei Reaktionstemperaturen von 30 bis 180°C, gegebenenfalls am Wasserabscheider, durchgeführt.

Dabei wird in der Regel ein äquivalentes Verhältnis von reaktionsfähigen Gruppen der blockierten Polyamine zu den reaktionsfähigen Gruppen des "Modifizierungsmittels" gewählt. Es ist jedoch auch möglich, die "Modifizierungsmittel" im Unterschuß, etwa unter Verwendung von 0,75 bis 0,99-fach äquivalenten Mengen, einzusetzen.

Durch die genannten Modifizierungsreaktionen zugängliche, als Komponente B) geeignete Polyamine sind beispielsweise Verbindungen der Formeln:

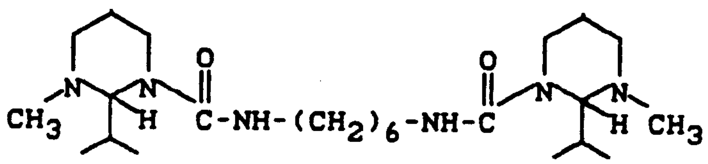

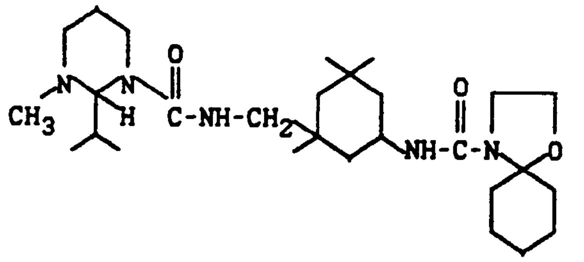

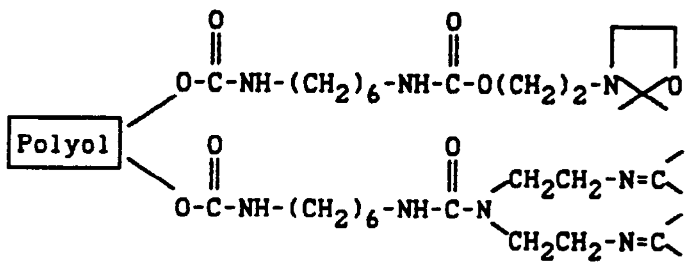

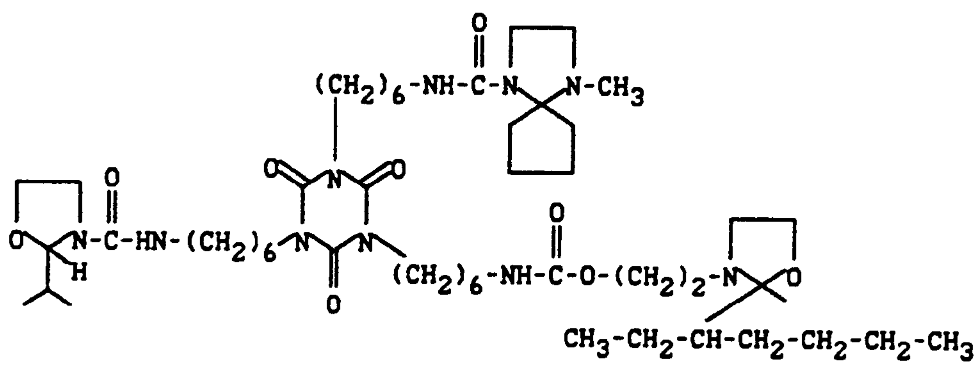

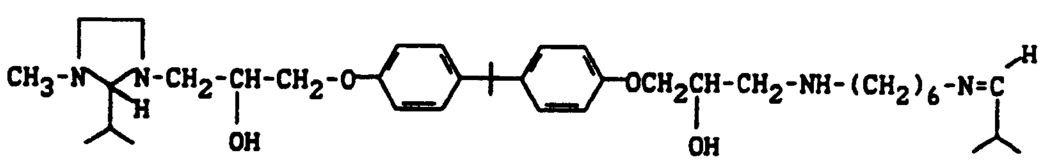

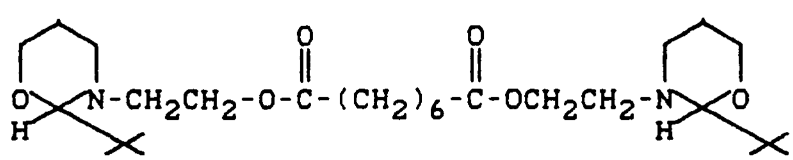

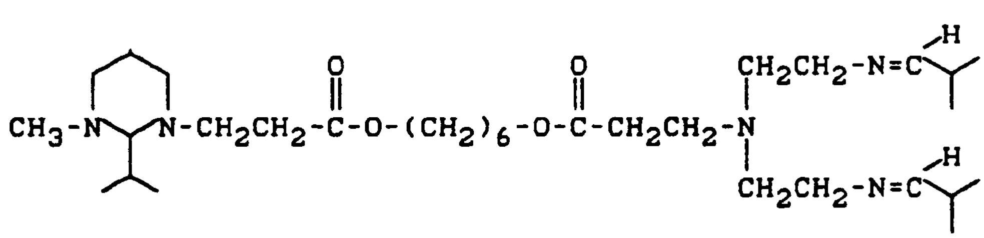

Die erfindungsgemäßen Bindemittel können neben den wesentlichen Komponenten A) und B) gegebenenfalls als weitere Komponenten C) Hilfs- und Zusatzmittel der unterschiedlichsten Art enthalten. Zu diesen gehören beispielsweise Lösungs- bzw. Verdünnungsmittel, Verlaufshilfsmittel, Antioxidantien, Füllstoffe, Pigmente und UV-Absorber.

Die Herstellung der erfindungsgemäßen Bindemittel bzw. Bindemittelkombinationen erfolgt dergestalt, daß man die Ausgangskomponenten A) und B), sowie gegebenenfalls die Hilfs- und Zusatzmittel C) miteinander vermischt. Im Falle der Mitverwendung von Lösungs- bzw. Verdünnungsmitteln als Komponente C) können diese bereits einer oder mehreren der Einzelkomponenten oder aber erst dem Gemisch aus den Komponenten A) und B) zugemischt werden. Insbesondere ist eine Ausführungsform denkbar, derzufolge die Lösungs- bzw. Verdünnungsmittel auch schon während der Herstellung einer oder mehrerer Ausgangskomponenten zugegen sind, wie dies z.B. vorstehend bei der Herstellung der Copolymerisate beschrieben worden ist. Die Lösungs- bzw. Verdünnungsmittel sollten weitgehend wasserfrei sein, um eine ausreichende Verarbeitungszeit der Gemische sicherzustellen. Lösungs- bzw. Verdünnungsmittel werden im allgemeinen in solchen Mengen mitverwendet, die zur Einstellung von geeigneten Verarbeitungsviskositäten der erfindungsgemäßen Kombinationen erforderlich sind. Der Lösungsmittelgehalt der der erfindungsgemäßen Verwendung zuzuführenden erfindungsgemäßen Bindemittel liegt im allgemeinen bei 10 bis 80 Gew.-%.

Durch Verwendung geeigneter, vergleichsweise niedermolekularer, Copolymerisate A) ist es jedoch auch prinzipiell möglich, den Lösungs- bzw. Verdünnungsmittelgehalt noch weiter zu reduzieren, bzw. auf die Mitverwendung dieser Hilfsmittel völlig zu verzichten.

Gemäß einer bevorzugten Ausführungsform werden als alleinige blockierte Polyamine Verbindungen B) eingesetzt, die keine in Abwesenheit von Feuchtigkeit gegenüber Isocyanatgruppen reaktionsfähige Gruppen aufweisen, und deren blockierte Aminogruppen ausschließlich aus Hexahydropyrimidin-, Tetrahydroimidazol-, Aldimin, Ketimin- und/oder Oxazolangruppen der genannten Art bestehen. Die bevorzugten, so hergestellten erfindungsgemäßen Kombinationen enthalten 40 bis 90 Gew.-Teile an Copolymerisaten A) und 10 bis 60 Gew.-Teile an Hexahydropyrimidin-, Tetrahydroimidazol, Ketimin-, Aldimin- bzw. Oxazolangruppen aufweisende Verbindungen B).

Die Mengenverhältnisse der Einzelkomponenten A) und B) werden im allgemeinen so gewählt, daß auf jede blockierte Aminogruppe der Komponente B) insgesamt 0,2 bis 8, vorzugsweise 0,5 bis 4 Isocyanatgruppen der Komponente A) entfallen. Im allgemeinen wird man einen höheren Überschuß an den letztgenannten Gruppen innerhalb der genannten Bereiche dann wählen, wenn die übrigen Komponenten reaktive Gruppen aufweisen, die in Abwesenheit von Feuchtigkeit mit Isocyanatgruppen reagieren. Hierzu gehören insbesondere primäre oder sekundäre Aminogruppen, die beispielsweise neben den blockierten Aminogruppen in der Komponente B) vorliegen können und auch alkoholische Hydroxylgruppen, die in der Komponente B) neben den blockierten Aminogruppen vorhanden sein können. Im allgemeinen sind jedoch alkoholische Hydroxylgruppen unter den vorherrschenden Bedingungen (Raumtemperatur) gegenüber tertiären Isocyanatgruppen weitgehend inert, so daß die Hydroxylgruppen nur dann in Betracht gezogen werden müssen, wenn sie in Form von schwerflüchtigen Alkoholen zum Einsatz gelangen, die bei der Verwendung der erfindungsgemäßen Kombinationen nicht verdunsten und, beispielsweise bei höheren Temperaturen während des Aushärtens von Beschichtungen als Reaktionspartner für die Komponente A) in Betracht kommen.

Insbesondere, wenn als Komponente B) solche Verbindungen eingesetzt werden, die neben den blockierten Aminogruppen freie primäre oder sekundäre Aminogruppen aufweisen, entstehen bei der Herstellung der erfindungsgemäßen Bindemittel durch Abmischen der Einzelkomponenten komplexe Gemische, in denen Harnstoffgruppen (durch Reaktion der Isocyanatgruppen mit Aminogruppen) vorliegen. Der Begriff "Bindemittel" umfaßt daher im Rahmen der Erfindung nicht nur, wie oben ausgeführt reine Gemische aus den Einzelkomponenten A) und B) sondern vielmehr auch Systeme, in denen neben den Einzelkomponenten derartige Umsetzungsprodukte vorliegen, bzw. die im wesentlichen ausschließlich aus derartigen Umsetzungsprodukten bestehen. Bei allen Varianten der Herstellung der erfindungsgemäßen Bindemittel durch Abmischen der Einzelkomponenten werden Art und Mengenverhältnisse dieser Einzelkomponenten vorzugsweise so gewählt, daß das Molverhältnis von tertiären Isocyanatgruppen zu blockierten Aminogruppen nach Beendigung der gegebenenfalls spontan ablaufenden Reaktion zwischen Isocyanatgruppen einerseits und primären bzw. sekundären Aminogruppen andererseits bei 0,2:1 bis 8:1 und insbesondere 0,5:1 bis 4:1 liegt, wobei auch hier ein Überschuß an Isocyanatgruppen in Betracht gezogen werden sollte, falls Einzelkomponenten mitverwendet werden, die alkoholische Hydroxylgruppen aufweisen, die bei der erfindungsgemäßen Verwendung jedoch neben den blockierten Aminogruppen als Reaktionspartner für die Isocyanatgruppen in Betracht kommen.

Unter "gegenüber tertiären Isocyanatgruppen reaktionsfähige Wasserstoffatome aufweisenden, blockierten Polyamine B)" sind im Rahmen der Erfindung im übrigen nicht nur solche blockierte Polyamine der genannten Art zu verstehen, die reaktionsfähige Wasserstoffatome in chemisch gebundener Form enthalten, sondern vielmehr auch solche, die im Gemisch mit überschüssigem, zu ihrer Herstellung eingesetztem Polyamin

bzw. Hydroxylamin vorliegen.

Bezüglich der Verwendbarkeit der erfindungsgemäßen Bindemittelkombinationen ist es weitgehend ohne Bedeutung, ob die gegebenenfalls spontan ablaufende Reaktion zwischen den Copolymerisaten A) und den gegenüber tertiären Isocyanatgruppen reaktionsfähigen Gruppen bereits vollständig zum Abschluß gekommen ist. Gewünschtenfalls ist es jedoch möglich, diese Reaktion vor der erfindungsgemäßen Verwendung durch kurzzeitiges Erhitzen auf 40 bis 100°C zum Abschluß zu bringen. Im übrigen erfolgt die Herstellung der erfindungsgemäßen Bindemittel vorzugsweise bei Raumtemperatur.

Die erfindungsgemäßen Bindemittel sind im allgemeinen (oftmals wegen des vorliegens von Lösungsmitteln) bei Raumtemperatur flüssig, sie sind in Abwesenheit von Wasser ausreichend lagerstabil und härten nach Applikation auf ein Substrat in Gegenwart von Luftfeuchtigkeit im allgemeinen rasch aus.

In der Regel werden bereits bei Raumtemperatur vernetzte Filme erhalten. Die an sich schon sehr rasche Aushärtung kann durch Trocknung bei höheren Temperaturen noch weiter beschleunigt werden. Dabei sind Temperaturen von 80 bis 130°C und Trocknungszeiten von 10 bis 30 Minuten vorteilhaft.

Bei Verwendung von besonders hydrolysestabilen blockierten Aminogruppen kann diese forcierte Trocknung bei höheren Temperaturen erforderlich sein, um das optimale Eigenschaftsbild zu erhalten.

Die die erfindungsgemäßen Bindemittel enthaltenden Beschichtungsmittel und Dichtmassen können die in der Lacktechnologie üblichen Hilfs- und Zusatzstoffe wie beispielsweise Pigmente, Füllstoffe, Verlaufshilfsmittel, Antioxidantien oder UV-Absorber enthalten.

Diese Hilfs- und Zusatzstoffe sollten möglichst wasserfrei sein und werden vorzugsweise bereits vor der Herstellung der Bindemittel den Ausgangskomponenten, im allgemeinen der Komponente A), einverleibt.

Die die erfindungsgemäßen Produkte als Bindemittel enthaltenden Lacke und Beschichtungsmassen weisen in Abwesenheit von Feuchtigkeit im allgemeinen eine Topfzeit von 3 bis 48 Stunden auf. Durch Auswahl geeigneter Reaktionspartner kann die Topfzeit jedoch beliebig nach oben oder unten korrigiert werden. Die Beschichtungsmittel (Lacke) und Dichtmassen können nach den üblichen Methoden, beispielsweise durch Spritzen, Streichen, Tauchen, Fluten, Gießen, Walzen, Spachteln auf beliebige, gegebenenfalls bereits vorbehandelte Untergründe wie z.B. Metall, Holz, Glas, Keramik, Stein, Beton, Kunststoffe, Textilien, Leder, Pappe oder Papier aufgetragen werden.

In den nachfolgenden Beispielen beziehen sich alle Prozentzahlen und -teile, falls nicht anders vermerkt, auf das Gewicht.

## Beispiele

I) Allgemeine Herstellvorschrift für die tertiäre Isocyanatgruppen aufweisenden olefinisch ungesättigten Verbindungen $a_1$ bis $a_3$

In einem 2-l-Reaktionsgefäß mit Rühr-, Kühl- und Heizvorrichtungen werden unter Stickstoffatmosphäre die Ausgangskomponenten vorgelegt und so lange bei 40°C gerührt, bis der theoretisch zu berechnende Isocyanatgehalt erreicht ist.

Die Zusammensetzungen der Ausgangskomponenten sind zusammen mit dem Isocyanatgehalt der erhaltenen isocyanatfunktionellen Monomeren in Tabelle 1 aufgeführt.

Tabelle 1 (Mengenangaben in g)

| Ausgangskomponenten | $a_1$ | $a_2$ | $a_3$ |
|---|---|---|---|
| 1-Isocyanato-1-methyl-4(3)-isocyanato-methylcyclohexan | 970 | 970 | 970 |
| Hydroxyethylacrylat | 580 | - | - |
| 4-Hydroxybutylacrylat | - | 720 | - |
| Hydroxyethylmethacrylat | - | - | 650 |
| 2,6-Di-tert.-butyl-4-methylphenol | 0,8 | 0,8 | 0,8 |
| Isocyanatgehalt (%) | 13,7 | 12,5 | 13,1 |
| Farbzahl (APHA) | 10 | 10 | 10 |

II) Allgemeine Herstellvorschrift für die tertiäre Isocyanatgruppen enthaltenden Copolymerisate $A_1$ bis $A_8$

In einem 3-l-Reaktionsgefäß mit Rühr-, Kühl- und Heizvorrichtungen wird Teil I vorgelegt und auf Reaktionstemaperatur aufgeheizt. Dann werden parallel Teil II (Zugabe über einen Zeitraum von insgesamt 2 Stunden) und Teil III (Zugabe über einen Zeitraum von insgesamt 2,5 Stunden) unter Stickstoffatmosphäre zudosiert. Anschließend wird 2 Stunden bei der Reaktionstemperatur nachgerührt.

Die Reaktionstemperaturen und die Zusammensetzungen der Teile I bis III sind in Tabelle 2, zusammen mit den Kenndaten der erhaltenen Copolymerisate aufgeführt.

**Tabelle 2** (Mengenangaben in g)

| Copolymerisate | $A_1$ | $A_2$ | $A_3$ | $A_4$ | $A_5$ | $A_6$ | $A_7$ | $A_8$ |
|---|---|---|---|---|---|---|---|---|
| **Teil I** | | | | | | | | |
| Xylol | 848 | | | 802 | | | 680 | 680 |
| Butylacetat | | 762 | 782 | | 684 | 568 | | |
| **Teil II** | | | | | | | | |
| Styrol | 390 | 280 | 214 | 216 | 60 | | | 304 |
| Ethylacrylat | | | | | | | 236 | |
| Butylacrylat | | 154 | 50 | | | | | 304 |
| Isobutylacrylat | | | | | 184 | | | |
| Methylmethacrylat | | 154 | 162 | 216 | | | 236 | |
| Ethylmethacrylat | | | | | | 380 | | |
| Butylmethacrylat | | | | | | | 236 | |
| Isobutylmethacrylat | | | | | | 252 | | |
| Cyclohexylmethacrylat | | | | | 436 | | | |
| 2-Ethylhexylmethacrylat | | | | | | 128 | | 184 |
| NCO-Monomer $a_1$ | 590 | 486 | 650 | 650 | | | | |
| NCO-Monomer $a_2$ | | | | | 488 | 504 | | |
| NCO-Monomer $a_3$ | | | | | | | 468 | 380 |
| **Teil III** | | | | | | | | |
| tert.-Butylperoctoat (70 %ig) | 58 | 64 | 64 | 56 | 72 | 76 | 68 | 68 |
| Xylol | 114 | | | 60 | | | 76 | 80 |
| Butylacetat | | 100 | 78 | | 76 | 92 | | |
| Polymerisationstemperatur, °C | 100 | 100 | 100 | 100 | 120 | 120 | 120 | 120 |
| Festgehalt, % | 50,5 | 55,3 | 55,4 | 55,9 | 59,7 | 64,5 | 59,9 | 60,4 |
| Viskosität 23°C, mPa.s | 1219 | 1417 | 1744 | 1370 | 4587 | 17150 | 4389 | 2725 |
| NCO-Gehalt der Lösung, % | 3,9 | 3,2 | 4,3 | 4,4 | 3,0 | 3,1 | 2,9 | 2,3 |
| Farbzahl, APHA | 30 | 40 | 30 | 20 | 40 | 10 | 20 | 10 |

III) Herstellung der zur Vernetzung befähigten Komponenten B)

$B_1$

In einem 2-l-Reaktionsgefäß mit Rühr-, Kühl- und Heizvorrichtung werden unter $N_2$-Atmosphäre 513 g Cyclohexan und 456 g Isobutyraldehyd vorgelegt. Bei 10°C (Eisbadkühlung) werden 529,8 g 1-Amino-3-methylaminopropan zugetropft, 1 Stunde bei 10°C gerührt und so lange auf Rückflußtemperatur erhitzt, bis kein Wasser mehr abgespalten wird. Anschließend werden Cyclohexan und überschüssiger Isobutyraldehyd abdestilliert und der Hexahydropyrimidinvernetzer $B_1$ erhalten.

$B_2$

In einem 3-l-Reaktionsgefäß mit Rühr-, Kühl- und Heizvorrichtung werden unter $N_2$-Atmosphäre 680 g Isophorondiamin, 1000 g Methylisobutylketon und 560 g Toluol so lange am Wasserabscheider unter Rückfluß erhitzt, bis die theoretische Wassermenge abgetrennt ist (144 g) bzw. bis sich kein Wasser mehr abscheidet. Anschließend werden Toluol und überschüssiges Methylisobutylketon abdestilliert und der Bisketiminvernetzer $B_2$ erhalten.

$B_3$

a) In einem 4-l-Reaktionsgefäß mit Rühr-, Kühl- und Heizvorrichtung werden unter $N_2$-Atmosphäre 1050 g Di-ethanolamin und 615 g Cyclohexan vorgelegt. Bei Raumtemperatur werden dann 1408 g 2-Ethylhexanal zugetropft, wobei die Temperatur langsam ansteigt. Es wird so lange bei Rückflußtemperatur gehalten, bis die Wasserabscheidung beendet ist. Anschließend werden Cyclohexan und überschüssiges 2-Ethylhexanal abdestilliert. Man erhält ein Oxazolan, die Vorstufe $B_3$a).

b) Herstellung von $B_3$

In einem Reaktionsgefäß mit Rühr-, Kühl- und Heizvorrichtungen werden unter $N_2$-Atmosphäre 200,6 g eines Isocyanuratgruppen aufweisenden Polyisocyanats auf Basis von Hexamethylendiisocyanat, welches im wesentlichen aus N,N',N''-Tris-(6-isocyanatohexyl)-isocyanurat besteht und 207 g Butylacetat vorgelegt und auf 60°C erwärmt. Nach dem Zutropfen von 286,7 g der Oxazolanvorstufe $B_3$a) wird noch 10 Stunden bei 70°C gehalten. Man erhält eine 70 %ige Lösung des im statistischen Mittel 3 Oxazolangruppen enthaltenden Vernetzers $B_3$.

IV) Herstellung der erfindungsgemäßen Bindemittelkombinationen

Die tertiäre Isocyanatgruppen enthaltenden Komponenten A) und die Polyaminkomponenten B) werden bei Raumtemperatur miteinander vermischt und gegebenenfalls durch Zugabe von weiteren Hilfsmitteln C) auf eine verarbeitungsfähige Viskosität eingestellt.

Die Filme werden mit einem Filmzieher auf Prüfbleche aufgezogen, wobei die Naßfilmstärke 180 µm beträgt. Die bei Raumtemperatur aufgezogenen und gelagerten Filme waren alle nach spätestens 60 Minuten klebfrei durchgetrocknet. Nach Alterung, d.h. 24 Stunden Raumtemperatur erhält man klare, vernetzte, lösungsmittelbeständige Filme mit hervorragender Filmmechanik und sehr guter Filmoptik.

Die angesetzten Lackmischungen wiesen durchweg eine Standzeit von mehreren Stunden auf.

In der folgenden Tabelle 3 werden die Zusammensetzungen der Bindemittelkombinationen und die Lösungsmittelfestigkeiten als Grad der Vernetzung aufgeführt.

Die Lösungsmittelfestigkeit wird durch einen Wischtest mit einem Methylisobutylketon (MIBK)-getränkten Wattebausch geprüft. Angegeben wird die Zahl der Doppelhübe, nach denen der Film ohne sichtbare Veränderung bleibt. Mehr als 200 Doppelhübe pro Film wurden nicht durchgeführt.

Tabelle 3

| Verwendungsbeispiel | 1 | 2 | 3 | 4 | 5 | 6 | 7 | 8 |
|---|---|---|---|---|---|---|---|---|
| Komponente A) | 50 g $A_1$ | 50 g $A_2$ | 50 g $A_3$ | 50 g $A_4$ | 50 g $A_5$ | 50 g $A_6$ | 50 g $A_7$ | 50 g $A_8$ |
| Komponente B) | 3,3 g $B_1$ | 2,7 g $B_1$ | 3,6 g $B_1$ | 3,7 g $B_1$ | 2,5 g $B_1$ | 2,6 g $B_1$ | 2,5 g $B_1$ | 2,0 g $B_1$ |
| Butylacetat | | 20 g | 20 g | | 20 g | 20 g | | |
| Xylol | 20 g | | | 20 g | | | 20 g | 20 g |
| Standzeit | >6 h | >8 h | >6 h | >6 h | >8 h | >8 h | >8 h | >8 h |
| MIBK-Wischtest nach 24 h Raumtemperatur Anzahl der Doppelhübe | >200 | >200 | >200 | >200 | >200 | >200 | >200 | 190 |
| Molverhältnis Isocyanatgruppen : Aminogruppen (nach hydrolytischer Freisetzung) | 1:1 | 1:1 | 1:1 | 1:1 | 1:1 | 1:1 | 1:1 | 1:1 |

**Tabelle 3**

| Verwendungsbeispiel | 9 | 10 | 11 | 12 | 13 | 14 | 15 | 16 |
|---|---|---|---|---|---|---|---|---|
| Komponente A) | 50 g $A_1$ | 50 g $A_2$ | 50 g $A_3$ | 50 g $A_4$ | 50 g $A_5$ | 50 g $A_6$ | 50 g $A_7$ | 50 g $A_8$ |
| Komponente B) | 7,8 g $B_2$ | 6,4 g $B_2$ | 8,5 g $B_2$ | 8,7 g $B_2$ | 6,0 g $B_2$ | 6,2 g $B_2$ | 5,8 g $B_2$ | 4,6 g $B_2$ |
| Butylacetat | | 20 g | 20 g | | 20 g | 20 g | | |
| Xylol | 20 g | | | 20 g | | | 20 g | 20 g |
| Standzeit | >6 h | >8 h | >6 h | >6 h | >8 h | >8 h | >8 h | >8 h |
| MIBK-Wischtest nach 24 h Raumtemperatur Anzahl der Doppelhübe | >200 | >200 | >200 | >200 | >200 | >200 | 200 | 180 |
| Molverhältnis Isocyanatgruppen : Aminogruppen (nach hydrolytischer Freisetzung) | 1:1 | 1:1 | 1:1 | 1:1 | 1:1 | 1:1 | 1:1 | 1:1 |

**Tabelle 3**

| Verwendungsbeispiel | 17 | 18 | 19 | 20 | 21 | 22 | 23 | 24 |
|---|---|---|---|---|---|---|---|---|
| Komponente A) | 50 g $A_1$ | 50 g $A_2$ | 50 g $A_3$ | 50 g $A_4$ | 50 g $A_5$ | 50 g $A_6$ | 50 g $A_7$ | 50 g $A_8$ |
| Komponente B) | 24 g $B_3$ | 20 g $B_3$ | 27 g $B_3$ | 27 g $B_3$ | 18,5 g $B_3$ | 19 g $B_3$ | 18 g $B_3$ | 14,5 g $B_3$ |
| Butylacetat | | 30 g | 35 g | | 30 g | 30 g | | |
| Xylol | 30 g | | | 35 g | | | 30 g | 25 g |
| Standzeit | >6 h | >8 h | >6 h | >6 h | >8 h | >8 h | >8 h | >8 h |
| MIBK-Wischtest nach 24 h Raumtemperatur Anzahl der Doppelhübe | >200 | >200 | >200 | >200 | >200 | >200 | >200 | >200 |
| Molverhältnis Isocyanatgruppen : Aminogruppen (nach hydrolytischer Freisetzung) | 1:1 | 1:1 | 1:1 | 1:1 | 1:1 | 1:1 | 1:1 | 1:1 |

## Patentansprüche

### Patentansprüche für folgende Vertragsstaaten : AT, BE, CH, DE, FR, GB, IT, LI, NL, SE

1. Olefinisch ungesättigte Verbindungen des Molekulargewichtsbereichs 252 bis 800, gekennzeichnet durch
   (i) einen Gehalt an Urethangruppen von 7 bis 24 Gew.-% und
   (ii) einen Gehalt an Isocyanatgruppen von 5 bis 17 Gew.-%, die an tertiäre Kohlenstoffatome gebunden sind, die ihrerseits Teil eines cycloaliphatischen Rings sind.
   wobei 1-Isocyanato-1-methyl-3-(2-allyloxy-ethoxycarbonylamino)-cyclohexan und 1-Isocyanato-1-methyl-3-(2-acryloxyethoxycarbonylamino)-cyclohexan ausgenommen sind.

2. Verfahren zur Herstellung von Isocyanatgruppen aufweisenden Copolymerisaten eines als Gewichtsmittel bestimmten Molekulargewichts von 1000 bis 100 000 durch radikalisch initiierte Copolymerisation von isocyanatgruppenfreien, olefinisch ungesättigten Monomeren mit olefinisch ungesättigten Monoisocyanaten in einer solchen Menge, daß in den resultierenden Copolymerisaten 0,1 bis 13 Gew.-%. an chemisch eingebauten Isocyanatgruppen bei einer mittleren NCO-Funktionalität der Copolymerisate von mindestens 2 vorliegen, dadurch gekennzeichnet, daß man als olefinisch ungesättigte Monoisocyanate Verbindungen gemäß Anspruch 1 verwendet.

3. Unter dem Einfluß von Feuchtigkeit aushärtbare Beschichtungsmittel oder Dichtmassen, die als Bindemittel ein Zweikomponenten-System aus einer Polyisocyanatkomponente und einer Polyaminkomponente mit blockierten Aminogruppen enthalten, dadurch gekennzeichnet, daß die Polyisocyanatkomponente aus gemäß Anspruchzerhaltenen Copolymerisaten besteht.

### Patentansprüche für folgenden Vertragsstaat : ES

1. Verfahren zur Herstellung von Isocyanatgruppen aufweisenden Copolymerisaten eines als Gewichtsmittel bestimmten Molekulargewichts von 1000 bis 100 000 durch radikalisch initiierte Copolymerisation von isocyanatgruppenfreien, olefinisch ungesättigten Monomeren mit olefinisch ungesättigten Monoisocyanaten in einer solchen Menge, daß in den resultierenden Copolymerisaten 0,1 bis 13 Gew.-% an chemisch eingebauten Isocyanatgruppen bei einer mittleren NCO-Funktionalität der Copolymerisate von mindestens 2 vorliegen, dadurch gekennzeichnet, daß man als olefinisch ungesättigte Monoisocyanate olefinisch ungesättigte Verbindungen des Molekulargewichtsbereichs 252 bis 800 verwendet, die
   (i) einen Gehalt an Urethangruppen von 7 bis 24 Gew.-% und
   (ii) einen Gehalt an Isocyanatgruppen von 5 bis 17 Gew.-%, die an tertiäre Kohlenstoffatome gebunden sind, die ihrerseits Teil eines cycloaliphatischen Rings sind,
   aufweisen, wobei 1-Isocyanato- 1-methyl-3-(2-allyloxy-ethoxycarbonylamino)-cyclohexan und 1-Isocyanato-1-methyl-3-(2-acryloxyethoxycarbonylamino)-cyclohexan ausgenommen sind.

2. Unter dem Einfluß von Feuchtigkeit aushärtbare Beschichtungsmittel oder Dichtmassen, die als Bindemittel ein Zweikomponenten-System aus einer Polyisocyanatkomponente und einer Polyaminkomponente mit blockierten Aminogruppen enthalten, dadurch gekennzeichnet, daß die Polyisocyanatkomponente aus gemäß Anspruch 1 erhaltenen Copolymerisaten besteht.

## Claims

### Claims for the following Contracting States : AT, BE, CH, DE, FR, GB, IT, LI, NL, SE

1. Olefinically unsaturated compounds having a molecular weight in the range from 252 to 800, characterized by
   (i) a content of 7 to 24% by weight of urethane groups and
   (ii) a content of 5 to 17% by weight of isocyanate groups attached to tertiary carbon atoms which are in turn part of a cycloaliphatic ring,
   1-isocyanato-1-methyl-3-(2-allyloxyethoxycarbonylamino)-cyclohexane and 1-isocyanato-1-methyl-3-(2-acryloxyethoxycarbonylamino)-cyclohexane being excluded.

2. A process for the production of copolymers containing isocyanate groups, which have a weight average

molecular weight of 1,000 to 100,000, by radical-initiated copolymerization of olefinically unsaturated monomers free from isocyanate groups with olefinically unsaturated monoisocyanates in such a quantity that the resulting copolymers contain from 0.1 to 13% by weight of chemically incorporated isocyanate groups for an average NCO functionality of the copolymers of at least 2, characterized in that unsaturated compounds of the type claimed in claim 1 are used as the olefinicaly unsaturated monoisocyanates.

3. Moisture-curing coating compositions or sealing compounds containing as binder a two-component system of a polyisocyanate component and a polyamine component containing blocked amino groups, characterized in that the polyisocyanate component consists of the copolymers obtained by the process claimed in claim 2.

**Claims for the following Contracting State : ES**

1. A process for the production of copolymers containing isocyanate groups, which have a weight average molecular weight of 1, 000 to 100, 000, by radical-initiated copolymerization of olefinically unsaturated monomers free from isocyanate groups with olefinically unsaturated monoisocyanates in such a quantity that the resulting copolymers contain from 0.1 to 13% by weight of chemically incorporated isocyanate groups for an average NCO functionality of the copolymers of at least 2, characterized in that olefinically unsaturated monoisocyanates having a molecular weight in the range from 252 to 800 are used which have
   (i) a content of 7 to 24% by weight of urethane groups and
   (ii) a content of 5 to 17% by weight of isocyanate groups attached to tertiary carbon atoms which are in turn part of a cycloaliphatic ring,
   1-isocyanato-1-methyl-3-(2-allyloxyethoxycarbonylamino)-cyclohexane and 1-isocyanato-1-methyl-3-(2-acryloxyethoxycarbonylamino) -cyclohexane being excluded.

2. Moisture-curing coating compositions or sealing compounds containing as binder a two-component system of a polyisocyanate component and a polyamine component containing blocked amino groups, characterized in that the polyisocyanate component consists of the copolymers obtained by the process claimed in claim 1.

**Revendications**

**Revendications pour les Etats contractants suivants : AT, BE, CH, DE, FR, GB, IT, LI, NL, SE**

1. Composés à insaturation oléfinique du domaine de poids moléculaire de 252 à 800, qui se caractérisent par
   (i) une teneur en groupes uréthanne de 7 à 24% en poids et
   (ii) une teneur en groupes isocyanate de 5 à 17% en poids, qui sont liés à des atomes de carbone tertiaires faisant à leur tour partie d'un noyau cycloaliphatique,
   le 1-isocyanato-1-méthyl-3-(2-allyloxyéthoxycarbonylamino)-cyclohexane et le 1-isocyanato-1-méthyl-3-(2-acryloxyéthoxycarbonylamino)-cyclohexane étant exclus.

2. Procédé pour la préparation de copolymères présentant des groupes isocyanate d'un poids moléculaire déterminé par moyenne pondérale de 1.000 à 100.000, par copolymérisation déclenchée par voie radicalaire de monomères à insaturation oléfinique exempts de groupes isocyanate avec des monoisocyanates à insaturation oléfinique en une quantité telle que, dans les copolymères résultants, on trouve de 0,1 à 13% en poids de groupes isocyanate incorporés par voie chimique, les copolymères présentant une fonctionnalité NCO moyenne d'au moins 2, caractérisé en ce qu'on utilise, comme monoisocyanates à insaturation oléfinique, des composés selon la revendication 1.

3. Agents d'enduction ou matières d'étanchéité durcissables sous l'action de l'humidité, qui contiennent, comme liant, un système à deux composants constitués par un composant de polyisocyanate et par un composant de polyamine comprenant des groupes amino bloqués, caractérisés en ce que le composant de polyisocyanate est constitué par des copolymères obtenus conformément à la revendication 2.

**Revendications pour l'Etat contractant suivant : ES**

1. Procédé pour la préparation de copolymères présentant des groupes isocyanate d'un poids moléculaire déterminé par moyenne pondérale de 1.000 à 100.000, par copolymérisation déclenchée par voie radicalaire de monomères à insaturation oléfinique exempts de groupes isocyanate avec des monoisocyanates à insaturation oléfinique en une quantité telle que, dans les copolymères résultants, on trouve de 0,1 à 13% en poids de groupes isocyanate incorporés par voie chimique, les copolymères présentant une fonctionnalité NCO moyenne d'au moins 2, caractérisé en ce qu'on utilise, comme monoisocyanates à insaturation oléfinique, des composés à insaturation oléfinique du domaine de poids moléculaire de 252 à 800, qui présentent
   (i) une teneur en groupes uréthanne de 7 à 24% en poids et
   (ii) une teneur en groupes isocyanate de 5 à 17% en poids, qui sont liés à des atomes de carbone tertiaires faisant à leur tour partie d'un noyau cycloaliphatique,
   le 1-isocyanato-1-méthyl-3-(2-allyloxyéthoxycarbonylamino)-cyclohexane et le 1-isocyanato-1-méthyl-3-(2-acryloxyéthoxycarbonylamino)-cyclohexane étant exclus.

2. Agents d'enduction ou matières d'étanchéité durcissables sous l'action de l'humidité, qui contiennent, comme liant, un système à deux composants constitué par un composant de polyisocyanate et par un composant de polyamine comprenant des groupes amino bloqués, caractérisés en ce que le composant de polyisocyanate est constitué par des copolymères obtenus conformément à la revendication 1.